# EUROPEAN PATENT SPECIFICATION

(11) **EP 2 100 132 B1**
(45) Date of publication and mention of the grant of the patent: **17.05.2017**
(21) Application number: 06716825.2
(22) Date of filing: 28.02.2006
(51) Int. Cl.: G01N 27/30

(54) **REFERENCE ELECTRODE FOR ELECTROANALYTICAL MEASUREMENTS, IN PARTICULAR FOR POTENTIOMETRIC MEASUREMENTS**
REFERENZELEKTRODE FÜR ELEKTROANALYTISCHE MESSUNGEN, INSBESONDERE POTENTIOMETRISCHE MESSUNGEN
ELECTRODE DE REFERENCE POUR MESURES ELECTROANALYTIQUES EN PARTICULIER POUR DES MESURES POTENTIOMETRIQUES

(30) Priority: 01.03.2005 PL 37327105
(43) Date of publication of application: 16.09.2009
(73) Proprietor: Thermo Fisher Scientific Oy, 01621 Vantaa (FI)
(72) Inventor: Lewenstam, Andrzej, 30 382 Krakow (PL); Blaz, Teresa, 30 611 Krakow (PL); Migdalski, Jan, 30 348 Krakow (PL); Duda, Ludomir, 05 506 Magdalenka (PL)
(74) Representative: Seppo Laine Oy
(86) International application number: PCT/PL2006/000014
(87) International publication number: WO 2006/093422

(56) References cited:
- EP-A- 1 193 495
- RO-B1- 104 155
- US-A- 4 563 263
- US-A- 4 908 117
- US-A1- 2004 256 227

## Description

The present invention concerns a reference electrode for electroanalytical measurements, in particular for potentiometric measurements in amphiprotic solvents, especially for the measurements in biological materials, body fluids, natural and tap waters, industrial waters and sewages, and other materials of ionic and nonionic amphiprotic character, including measurements in biomedical samples.

In the potentiometric measurements the signal is measured as a difference of electrical potentials between the indicator electrode, which is sensitive for determined substance (analyte), and the reference electrode, which is not sensitive. The reference electrode used in electroanalytical measurements, including the potentiometric measurements, should be characterized with constant or ignorable potential changes over the measurement time, that is independent from matrices containing analytes and their concentration changes, in particular the solutions used for calibration.

In the literature there are described various reference electrodes, including classical conventional electrodes, such as standard hydrogen electrode, calomel electrode and silver chloride electrode. Among fundamental drawbacks of these electrodes are: the necessity of usage of the highly concentrated solutions or gases, the need of periodic maintenance, uselessness in modern highly automatic analytical techniques of measurement especially these with a high throughput, difficulties with miniaturization and down-scaling for nanotechnological applications. To avoid these hindrances several auxiliary solutions are applied. One of them is the application of electrolytic bridge for the measurements in the open liquid junction regime. The former concept provides additional electrical potential source, so-called diffusion potential, which must be compensated, the latter solution demands additional resolutions enforcing flow, for instance additional peristaltic pump. Another solution is dispersing the electrolytes of the bridge in non-conducting polymer [T.Sokalski, M.Maj-Zurawska, A.Hulanicki, A.Lewenstam, Electroanalysis, 1999,9,632-636; "Optimization of a reference electrode with constrained liquid junction for the measurements of ions"]. Yet another one is the addition of indicating ion into the solution measured, which role is to stabilize the potential the reference electrode used, without disturbing the signal of the indicator electrode. This possibility is referred to as pseudoreference electrode [D.Diamond, E.McEnroe, A.Lewenstam, Electroanalysis, 1994,6,962-971; "Evaluation of a new reference electrode junction material for ISEs"].

EP 1193 495 discloses a polymeric reference electrode membrane comprising porous polymer or a hydrophilic plasticizer or lipophilic polymer. It provides shortened preconditioning time and exhorted storage lifetime.

In the measurement systems the ion-selective electrodes (ISE) with electroactive layer are made of the solid crystalline membranes, the plastic membranes or other materials enriched with dispersed in their bulks chemical systems, selected and aimed to increase the selectivity and to broaden the applicability or to enable the use of conducting polymers (CP). These layers may be cast on the electronically conducting substrate, including glassy carbon, conducting glass, conducting polymers or metallic platform.

It is known that the conducting polymers exhibit mixed electrical conductivity, ie. ionic and electronic conductivity, which may be modified by doping with co-ions. In dependence on the kind of doping ion the CP films can exhibit ionic sensitivity, for cation or anion concentration changes in the studied solution, bathing the CP film.

It is also known that the amphiprotic solution ions, such as water, defined acid-base pairs stabilize the concentration of hydrogen ions, forming pH buffer. Some of the pH buffers can have additional chemical properties that enable their effective application in multicomponent analyses. Namely, components of these buffers do not bind the ions present in biological samples and are not engaged in side reactions with the components of matrix, for instance do not react with gases. These properties are characteristic for so-called biological pH buffers.

The purpose of the present invention is to obtain the active material of the reference electrode which behaves as a pH buffer, therefore which means that is not sensitive for pH changes in the range of buffering and does not change significantly its electrical potential in contact with the analyzed solutions.According to claim 1, the reference electrode for electroanalytical measurements, in particular for potentiometric measurements, is characterized by having solid electroactive conducting layer comprising dispersed in the layer molecules of chemical compounds containing groups constitutive for pH buffers, namely biological buffers. As for the molecules dispersed in the conducting layer, they belong to the group of compounds containing sulfonic group. It is preferred if the compounds containing the sulfonic group form biological buffers, in particular such as:
2-Morpholineoethanesulfonic acid,
3- Morpholineopropanesulfonic acid,
piperazine-1,4-bis(2-ethanesulfonic) acid,
2-[4-(2-hydroxyethyl)-1-piperazine]ethanesulfonic acid,
N-[Tris(hydroxymethyl)methyl]-2-aminoethanesulfonic acid,
1-Hydroxy-4-sulfobenzoic acid.

In the preferred realizations of the reference electrode the conducting layer is formed by conducting polymer, and the molecules of the compounds having the groups constitutive for pH buffers are dispersed in the phase of the conducting polymer. It is advantageous if the conducting polymer is poly(pyrrole) or its derivate, polythiophene or its derivate, polyaaniline or its derivate, or a mixture of just listed polymers with other electrically conducting compounds.

In another realization of the reference electrode the conducting layer is formed by the skeleton of modified carbon fibers, wherein the molecules of the compounds having pH constitutive groups are bound with the carbon skeleton of the conducting layer.

It is advantageous if the thickness of the conducting layer of the reference electrode is in the range 0.4 + 30 micrometers, is deposited on the solid conducting substrate, and additionally is stabilized by conditioning in the solutions of the compounds having the same pH constitutive groups.

According to the invention, the electrode described has a solid conducting layer (sufficient to enable co-operation with ion-meter), which may be produced electrochemically on the conducting substrate by electropolymerization from the solution containing the monomer of the polymer and modifiers taken in appropriate concentrations, which are the pH buffers, in particular biological buffers, or a conducting layer made chemically by dissolving polymer and the modifiers in a properly selected solvent, after its subsequent evaporation from the substrate. These buffers modify the polymer phase due to formation of electrostatic bounds with the oppositely charged active groups of the polymer core, due to formation of the covalent bound with properly modified fragments of the conducting polymer or due to mechanical dispersion in the polymer phase. The electroactive conducting layer may be obtained in the form of film in water or non-water solutions containing monomer, dimmer or n-mer of the polymer from the group of polypyrrole or its derivate, polythiophene or its derivate, polyaniline or its derivate, or their mixtures that are useful form the perspective of the goal of this invention. In the case of depositing the film by electropolymerization appropriate magnitudes of electrical current and potential are applied, either eletrodepositing takes place under constant (controlled) potential, or constant current or controlled potential or current, varied with time. In this way the films of controlled thickness are deposited at the substrate. According to the invention presented the film of the thickness 0.4-30 micrometers are optimal. In order to amplify the property of pH buffering the films is submitted for conditioning in the buffer of appropriate pH. By conditioning in the solution containing the same constitutive groups in the buffer and in the electroactive layer the maximum inhibition of pH sensitivity is obtained.

The electroactive conducting layer may be deposited using the base of durably modified carbon fibers, wherein the molecules of the compounds having pH constitutive groups ought to be bound with this layer or incorporated in the layer. Modification of graphite, carbon fibers or fiber fabric is based on oxidizing in liquid or gaseous oxidant. For instance in concentrated nitrogen acid or ozone and producing variousfunctional groups containing oxygen with acidic or basic properties (for instance carboxylic groups, hydroxyl phenyl groups, carbonyl, chinoidyl or lactone groups). The oxygen functional groups may exhibit basic or acidic character. These groups have ability for ion-exchange or adsorption of ions of acidic or basic character, they may form donor-acceptor associates, including the molecules of the compounds having pH buffer constitutive groups. The electrodes in this realization may be useful in special applications.

In the course of the measurements in which the reference electrodes invented and described herein are applied, the amphiprotic solutions containing ions that are the subject of determination, the solutions of pH range determined by the chemical properties of the film and its buffering properties can be used, alike as in the case of pH buffers in water solutions. However, buffering of pH of the solution studied is not indispensable.

The measurement with the reference electrode being a subject of this invention has several advantages in comparison with presently used setups: the electrode works both in watery and non-water media, it is maintenance-free and may be stored: "dry", it has good durability and long period of proper operation, it may be miniaturized and processed, i.e. it allows achieving desirable shape of the electroactive surface. Moreover, it does not demand applying an electrolytic bridge in a measuring system.

The chemical names of the commercial biological buffers MES, MOPS, PIPES, HEPES, TES, SSA described herein are defined as follows:
MES: C₆H₁₃NO₄S 2-Morpholineoethanesulfonic acid.
MOPS: C₇H₁₅NO₄S 3-[N-morpholino]propanesulfonic acid; 3-Morpholineopropanesulfonic acid.
PIPES: C₈H₁₈N₂O₆S₂ Piperazine-1,4-bis[2-ethanesulfonic] acid; piperazine-1,4-bis(2-ethanesulfonic) acid.
HEPES: C₈H₁₈N₂O₄S 2-[4-(2-Hydroxyethyl)-1-piperazinyl]-ethanesulfonic acid; 2-[4-(2-hydroxyethyl)-1-piperazine]ethanesulfonic acid.
TES C₆H₁₅NO₆S N-[Tris(hydroxymethyl)methyl]-2-aminoethanesulfonic acid.
SSA: C₇H₆O₆S H₂O 1-Hydroxy-4-sulfobenzoic acid.
Additionally the following abbreviations are used:
EDOT: 3,4-ethylenedioxythiophene; 3,4 etylenodioksytiofen, monomer;
PEDOT: the polymer obtained by polymerization of EDOT;
PPy lub PMPy: polypyrrole and poly(1-metylopyrrole), the polymers obtained by polymerization of the monomer of respective compounds.
For simplicity the unit mol dm⁻³ is substituted by M.

The electrode described in the invention is more closely explained in the example of its construction and application as illustrated by the accompanying drawings, where fig. 1 shows the reference electrode in a form of rod with front-cast electroactive layer in a cross-cut view; fig. 2a shows the inverse calibration curve for PEDOT-SSA film in the pH range 3-6; fig. 2b shows the inverse calibration curve for PEDOT-SSA film in the pH range 6-10; fig. 3a shows the inverse calibration curve for PEDOT-MES film in the pH range 5,6-6,5; fig. 3b shows the reverse calibration curve for PEDOT-MES film in the pH range 6,5-5,6; fig. 4a shows the inverse calibration curve for MPPy-MOPS film in the pH range 6,70-7,55; fig. 4b shows the reverse calibration curve for MPPy-MOPS film in the pH range 7,55-6,70; fig.5 illustrates the lack of sensitivity of PEDOT-SSA films made ready for use in the pH range 6-10 for pH changes and for the changes of dissolved oxygen; fig.6 shows the changes of the potential for the films PEDOT-SSA i PEDOT-MES during titration of the mixture of HCl+NaCl with pH = 5,7, by means of diluted NaOH; fig.7 shows, selected for comparison, titration curves of sulphates with Pb(II) ions in water-methanol medium.

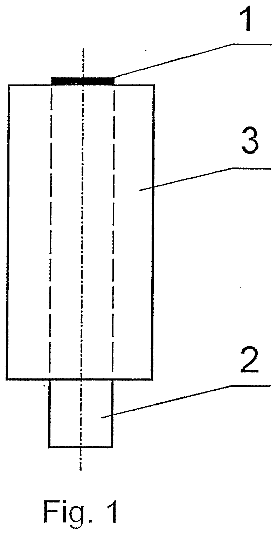

The exemplary electrode show in fig. 1 is made of a solid electroactive conducting layer 1 deposited on the conducting substrate in form of a rod 2 placed in protecting body made of plastic 3. The layer deposited at the conducting substrate 1 constitutes the reference film of 0.4-30 micrometers thick. The cross-surface of the rod 2 is 0.03-0.07 cm².

To get made the reference electrode with conducting layer made of conducting polymer, the rod 2, made of gold (Au) or glassy carbon (GC), after cleaning and washing with water, was place in the solution, from which the electropolymerization was done, as a disk electrode working in the system with auxiliary Pt electrode of the surface ca. 2 cm² and the reference electrode Ag/AgCl/3M KCl. The reference electrode was connected by means of a bridge filled with 0.1-1M solution of 1 hydroxy-4-sulfobenzoic acid.

For preparation of the solutions, in which the electropolymerization was performed, following biological buffers were used: , MOPS, PIPES, HEPES, TES, SSA and selected monomers of conducting polymers. The sollution was saturated with argon and the argon flow was maintained also during deposition of the film. The film was deposited from the solution at the electrode substrate through potential cycling with a scan rate 20 mV/s or potentiostatically.

After the depositing process was finished, the film taken away from the solution and rinsed with water, was left for drying. In order to enhance pH buffering property of the film that constitutes electroactive layer of the reference electrode was stabilized by conditioning in the buffer having the same constitutive pH buffer groups.

The conditions of depositing were as follows:

### Example 1

PEDOT-SSA film was deposited from the solution containing 0,01M EDOT and 3M or 0,1M 1-hydroksy-4-sulfobenzoic acid (SSA) during potential cycling in the range from 500 to 850-900 mV performed purposely in such a way that the density of current in the 20^{th} cycle was in the range 1,1-1,7 mA/cm².

### Example 2

PEDOT-MES film was deposited potentiostatically from the solution containing 0,01M EDOT and 0,4 M or 1,33 M MES. The deposition process was conducted under constant potential +1050 mV during 800 - 1600 seconds.

### Example 3

PEDOT-PIPES film was deposited potentiostatically from the solution containing 0,01M EDOT and PIPES (saturated solution, approx. 0,005 M). The deposition process was conducted under constant potential +1050 mV during 600 - 1200 seconds.

### Example 4

PMPy-MES film was deposited potentiostatically from the solution containing 0,1MPy and 0,4 M or 1,33 M MES. The deposition process was conducted under constant potential +750 mV during 800 - 1600 seconds.

### Example 5

PMPy-MOPS film was deposited potentiostatically from the solution containing 0,1MPy and 0,8 M of MOPS. The deposition process was conducted under constant potential +950 mV during 800 - 1600 seconds.

### Example 6

PMPy-PIPES film was deposited potentiostatically from the saturated solution of PIPES, (approx. 0,005 M) and 0,1 M MPy. The deposition process was conducted under constant potential +900 mV during 600 - 1200 seconds.

In analogous conditions the films containing another biological buffers were deposited and made ready for measurements. Te electrochemical properties of thus made reference electrodes were comparable (stability of the potenatial vs pH, influence of redox, life time). Between the measurements the electrodes were immersed in the appropriate pH buffer. During 8 months of use the worsening of their functional properties was not observed.

Some of the characteristics and aspects of practical application of selected reference electrodes in electroanalytical measurements, including the determination of sulphates by titration in water-methanol medium are presented below.

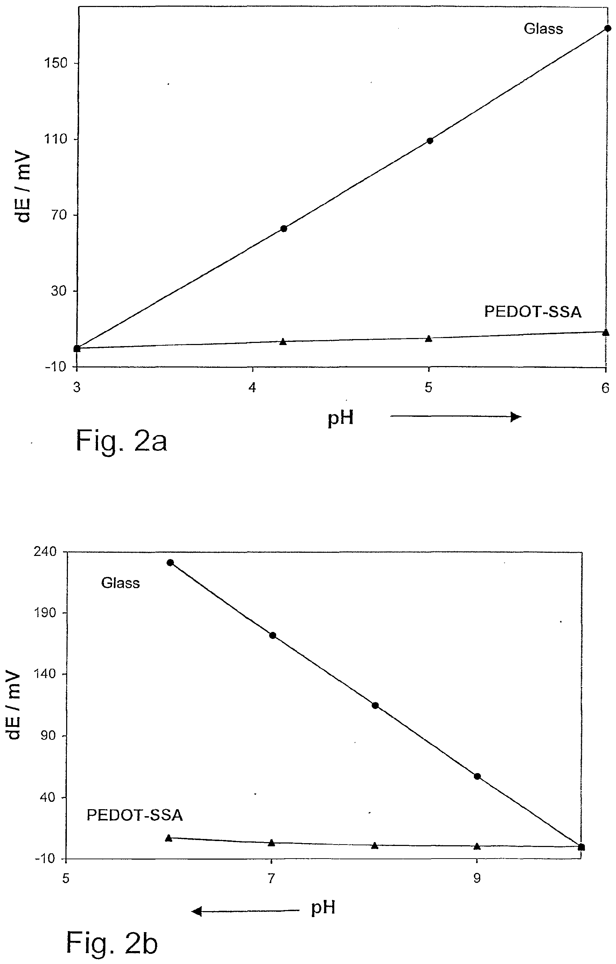

In the fig.2a and 2b the exemplary calibration curves in the universal pH buffers for the films PEDOT-SSA, prepared for operation in pH range 3-6 (fig.2a) or in the pH range 6-10 (fig.2b) are shown. They confirm reduced pH sensitivity of the films. For comparison the potential changes of pH glass electrode are shown.

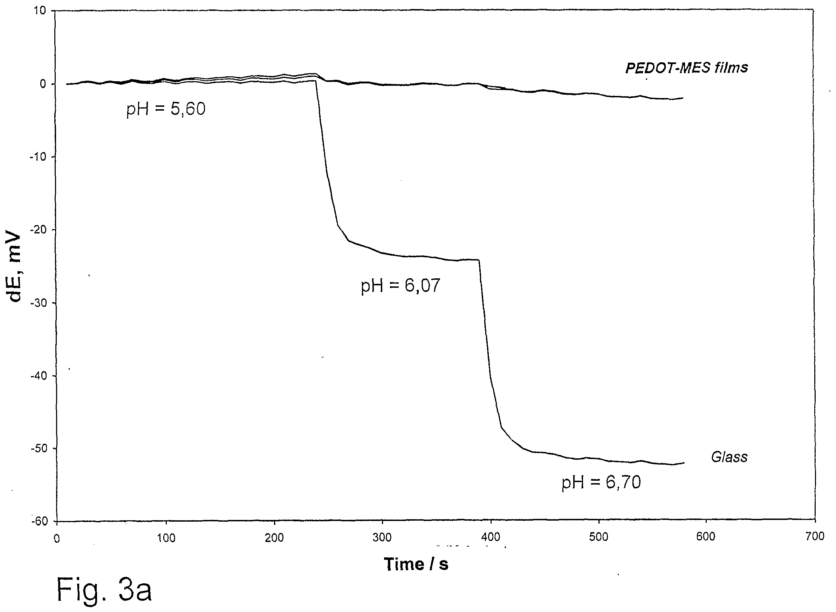

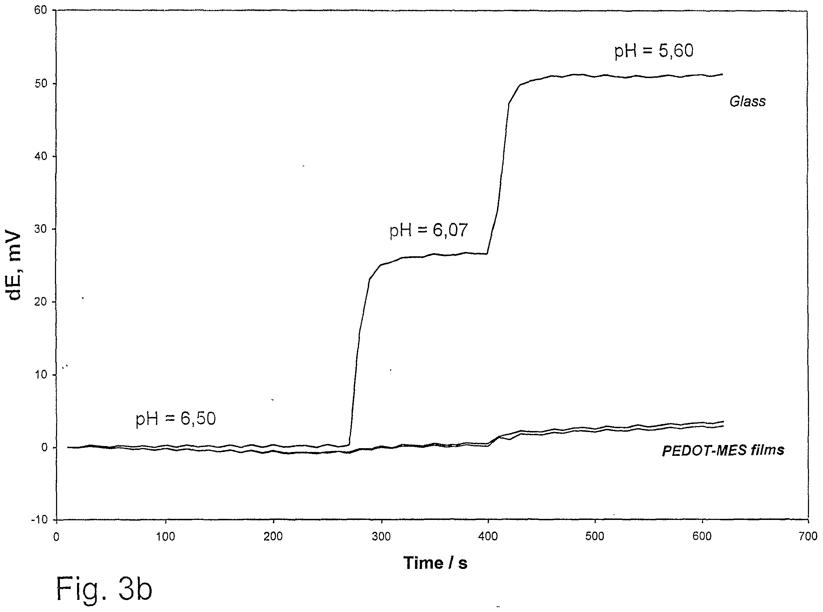

The exemplary calibration curves, shown in Figs 3a and 3b, made in the universal pH buffers for the films PEDOT-MES as well confirm the reduced pH sensitivity in the range of pH buffering by MES. Fig. 3a presents the calibration curve in the pH range 5.6-6.5, while fig. 3b the inverse calibration in the pH range 6.5-5.6. For comparison the potential changes of pH glass electrode are shown.

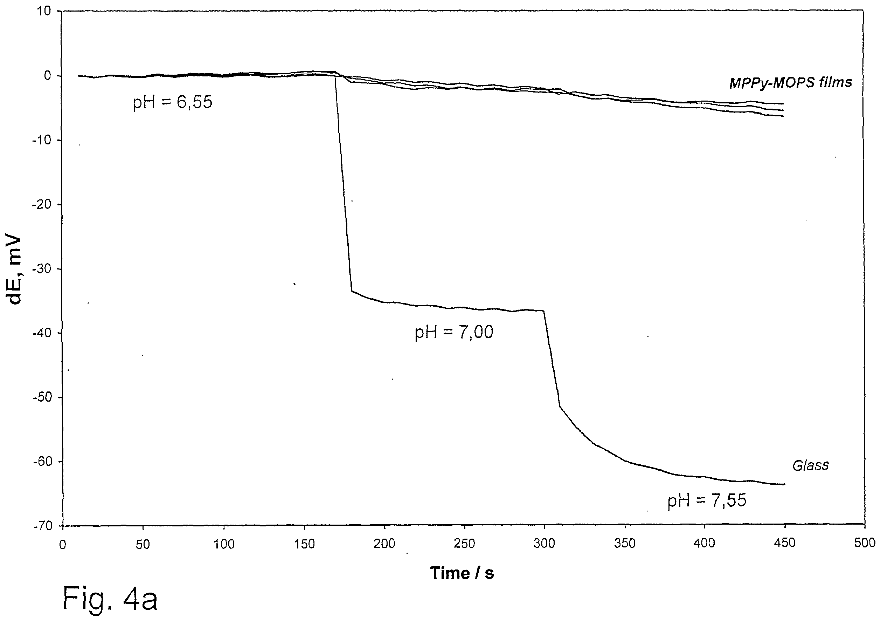

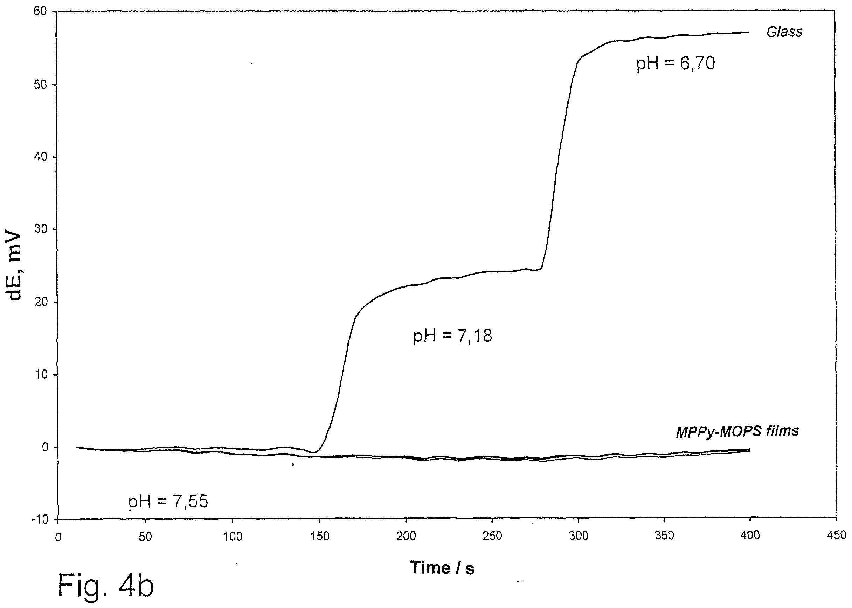

The exemplary calibration curves of the MPPy-MOPS films made in the buffering range of MOPS, confirmed also reduced pH sensitivity. Fig. 4a shows the calibration curve in the pH range 7,55 - 6,70, while in fig. 4b inversed calibration in the pH range 7,55 - 6,70 is presented. For comparison potential changes of the glass electrode are shown.

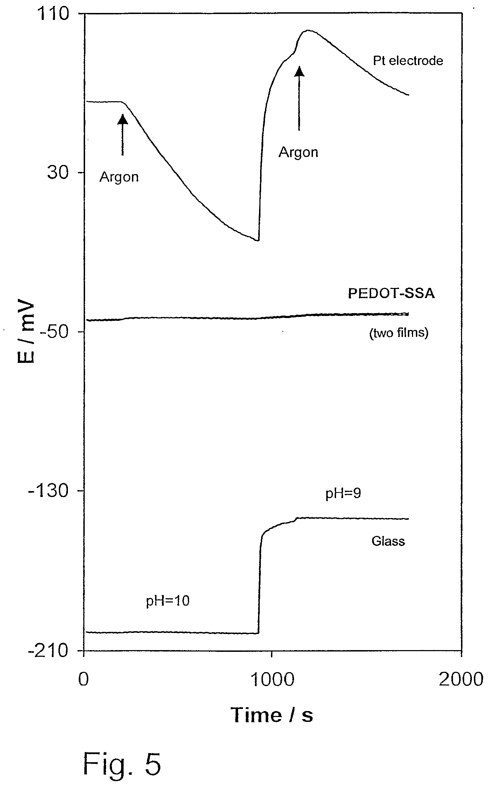

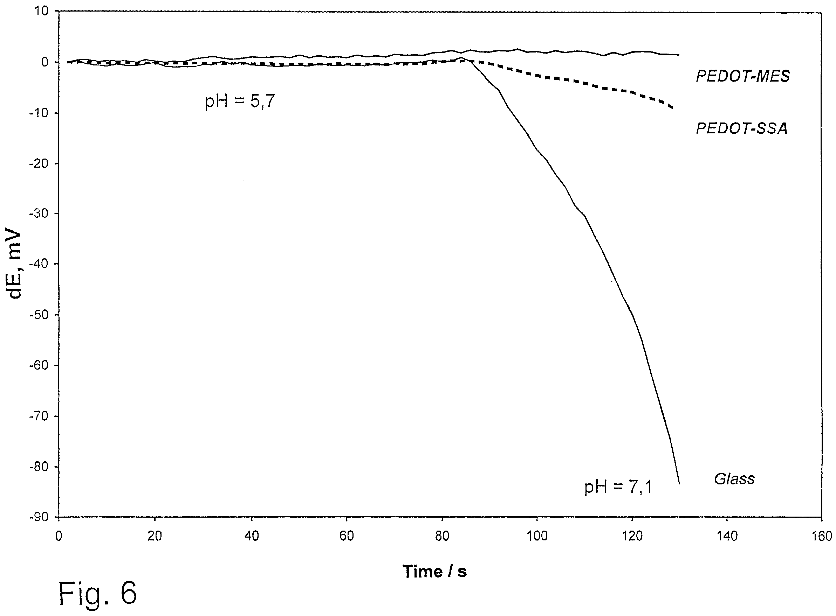

Fig. 5 illustrates reduced sensitivity of PEDOT-SSA films prepared for working in the pH range 6-10 for pH changes and for the changes of dissolved oxygen. For comparison the potential changes of a glass electrode and platinum redox electrode are shown. In the places indicated by the arrows saturating of the solutions studied with argon was initiated. During measurements the films were characteristic of a very good potential stability both during changes of pH and the changes of the concentration of oxygen dissolved in the solution. In fig. 6 the potential changes of PEDOT-SSA and PEDOT-MES in the course of titration of the HCl + NaCl mixture, with initial pH=5,7, with NaOH are shown. For comparison the potential changes of a glass electrode are shown. The range of pH changes includes the MES buffering range, but does not include the buffering range of SSA.

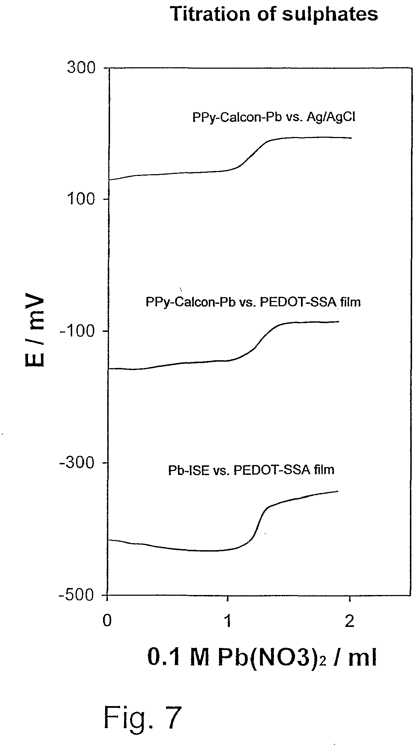

In fig.7 the titration curves of sulphates with Pb(II) ions in water-methanol medium are shown while applying:
a) commercial ion-selective lead electrode with crystalline membrane and the reference film PEDOT-SSA (the curve Pb-ISE vs. the film PEDOT-SSA),
b) the film PPy- Calcon made sensitive for Pb(II) ions and the conventional reference electrode Ag/AgCl/3MKCl with double bridge (the curve PPy-Calcon-Pb vs. Ag/AgCl),
c) the film PPy-Calcon made sensitive for Pb(II) ions and the reference film PEDOT-SSA (the curve PPy-Calcon-Pb vs. the film PEDOT-SSA).

In another realization of the reference electrode the electroactive conducting layer 1 constitutes the skeleton of modified carbon fibers. In order to obtain the conducting layer bound with carbon matrix the compounds having pH buffers constitutive groups, modified carbon fibers were grinded, mixed with proper compound having pH buffers constitutive groups and delicately heated under increased pressure. The powder obtained was introduced into the solution of polyvinyl chloride (PCV) in suitable solvent (e.g. in tetrahydrofurene THF) or other binding matrix, and the suspension obtained was cast on the conducting substrate made of the rod 2 and left until complete evaporation of the solvent. In order to enhance the pH buffering property the film constituting electroactive layer was stabilized by conditioning in the pH buffer having the same constitutive pH buffer groups.

The reference electrode made according to the invention may be used in analyses and measurements in various electroanalytical methods, in particular in potentiometric measurements, in amphiprotic media (solvents, vapors, gases, solid bodies), in the buffering range resulting from the properties of electroactive layer. It is especially useful in direct potentiometry and potentiometric titrations, in particular in the measuring system with all ion-selective electrodes. It may be applied as well in a system with classical measuring electrodes. And in the system with several such electrodes.

The reference electrode made according to the invention behaves in the solutions as a pH buffer. With increasing quantity of a modifier and the active groups in the conducting polymer the sensitivity of the electrode for changes of pH in the range resulting from the properties of pH buffering groups decreases. Worsening of measurement precision may appear in the case of redox influence as well as on the limiting ranges of buffering. The electrode as shown in the invention is suitable for performing analyses of biological materials, biological fluids (blood, urine), natural waters, industrial waters, sewages, and other substances of ionic character. The assembles of such electrodes can be as well applied for the measurement of potential difference, electrocardiography, encephalography, and similar applications.

## Claims

1. Reference electrode for electroanalytical measurements, in particular potentiometric measurements, having a solid electroactive conducting layer (1) comprising dispersed in the layer molecules of chemical compounds containing groups constitutive for pH buffers, **characterized in that** said chemical compounds are biological buffers containing sulfonic groups.

2. The electrode according to claim 1, wherein said biological buffers containing sulfonic groups are selected from the group consisting of:
2-Morpholineoethanesulfonic acid,
3- Morpholineopropanesulfonic acid,
Piperazine-1,4-bis(2-ethanesulfonic) acid,
2-[4-(2-hydroxyethyl)-1-piperazine]ethanesulfonic acid,
N-[Tris(hydroxymethyl)methyl]-2-aminoethanesulfonic acid,
1-hydroxy-4-sulfobenzoic acid.

3. The electrode according to claim 1, wherein the conducting layer (1) is a conducting polymer, and the molecules of the compounds containing pH buffer constitutive groups are dispersed in the phase of conducting polymer.

4. The electrode according to claim 3, wherein the conducting polymer is poly(pyrrole) or poly(pyrrole) derivates, polytiophene or polytiophene derivates, polyaniline or polyaniline derivates or a mixture of these polymers with other electroconducting compounds.

5. The electrode according to claim 1, wherein the conducting layer (1) is constituted of carbon fibers or tubes, and the compounds containing pH buffer constitutive groups are bound with carbon chain of conducting layer.

6. The electrode according to claim 1, wherein the conducting layer (1) is 0.4 - 30 micrometer thick.

7. The electrode according to claim 1, wherein the conducting layer (1) is deposited on solid conducting substrate (2).

8. The electrode according to claim 1, wherein the conducting layer (1) is stabilized by conditioning in solutions containing the compounds with the constitutive groups of pH buffers.

## Patentansprüche

1. Referenzelektrode für elektroanalytische Messungen, insbesondere potentiometrische Messungen, mit einer festen elektroaktiven leitenden Schicht (1), umfassend, in der Schicht dispergiert, Moleküle chemischer Verbindungen, welche Gruppen enthalten, die pH-Puffer bilden, **dadurch gekennzeichnet, dass** die chemischen Verbindungen biologische Puffer sind, die Sulfon-Gruppen enthalten.

2. Elektrode nach Anspruch 1, wobei die biologischen Puffer, die Sulfon-Gruppen enthalten, ausgewählt sind aus der Gruppe bestehend aus:
2-Morpholinoethansulfonsäure,
3-Morpholinopropansulfonsäure,
Piperazin-1,4-bis-(2-ethansulfon)-säure,
2-[4-(2-Hydroxyethyl)-1-piperazin)-ethansulfonsäure,
N-[Tris-(hydroxymethyl)-methyl]-2-aminoethansulfonsure,
1-Hydroxy-4-sulfobenzoesäure.

3. Elektrode nach Anspruch 1, wobei die leitende Schicht (1) ein leitendes Polymer ist, und die Moleküle der Verbindungen, welche Gruppen enthalten, die pH-Puffer bilden, in der Phase des leitenden Polymers dispergiert sind.

4. Elektrode nach Anspruch 3, wobei das leitende Polymer Poly-(pyrrol) oder Poly-(pyrrol)-Derivate, Polythiophen oder Polythiophen-Derivate, Polyanilin oder Polyanilin-Derivate oder eine Mischung dieser Polymere mit anderen elektroleitenden Verbindungen sind.

5. Elektrode nach Anspruch 1, wobei die leitende Schicht (1) aus Kohlefasern oder Kohlenstoffröhrchen besteht, und die Verbindungen, welche Gruppen enthalten, die pH-Puffer bilden, mit der Kohlenstoffkette der leitenden Schicht gebunden sind.

6. Elektrode nach Anspruch 1, wobei die leitende Schicht (1) 0,4 bis 30 Mikrometer dick ist.

7. Elektrode nach Anspruch 1, wobei die leitende Schicht (1) auf einem festen leitenden Substrat (2) abgeschieden ist.

8. Elektrode nach Anspruch 1, wobei die leitende Schicht (1) durch Konditionieren in Lösungen, welche die Verbindungen mit den Gruppen enthalten, die pH-Puffer bilden, stabilisiert ist.

## Revendications

1. Electrode de référence pour mesures électro-analytiques, en particulier pour mesures potentiométriques, ayant une couche conductrice électro-active solide (1) comprenant, dispersées dans la couche, des molécules de composés chimiques contenant des groupements constitutifs pour des tampons de pH, **caractérisé en ce que** lesdits composés chimiques sont des tampons biologiques contenant des groupements sulfoniques.

2. Electrode selon la revendication 1, dans laquelle lesdits tampons biologiques contenant des groupements sulfoniques sont choisis dans le groupe constitué des suivants :
acide 2-morpholinoéthanesulfonique,
acide 3-morpholinopropanesulfonique,
acide pipérazine-1,4-bis(2-éthanesulfonique),
acide 2-[4-(2-hydroxyéthyl)-1-pipérazine]éthanesulfonique,
acide N-[tris(hydroxyméthyl)méthyl]-2-aminoéthanesulfonique,
acide 1-hydroxy-4-sulfobenzoïque.

3. Electrode selon la revendication 1, dans laquelle la couche conductrice (1) est un polymère conducteur, et les molécules des composés contenant des groupements constitutifs de tampons de pH sont dispersées dans la phase du polymère conducteur.

4. Electrode selon la revendication 3, dans laquelle le polymère conducteur est formé de poly(pyrrole) ou de dérivés de poly(pyrrole), de polythiophène ou de dérivés de polythiophène, de polyaniline ou de dérivés de polyaniline ou d'un mélange de ces polymères avec d'autres composés électroconducteurs.

5. Electrode selon la revendication 1, dans laquelle la couche conductrice (1) est constituée de fibres ou de tubes de carbone, et les composés contenant des groupements constitutifs de tampons de pH sont liés à la chaîne de carbone de la couche conductrice.

6. Electrode selon la revendication 1, dans laquelle la couche conductrice (1) a une épaisseur de 0,4 à 30 micromètres.

7. Electrode selon la revendication 1, dans laquelle la couche conductrice (1) est déposée sur un substrat conducteur solide (2).

8. Electrode selon la revendication 1, dans laquelle la couche conductrice (1) est stabilisée par conditionnement dans des solutions contenant les composés avec les groupements constitutifs de tampons de pH.
